# EUROPEAN PATENT APPLICATION

(11) **EP 4 775 958 A1**
(43) Date of publication of application: **15.07.2026**
(21) Application number: 26150009.4
(22) Date of filing: 02.01.2026
(51) Int. Cl.: G01N 21/31, G01N 21/39, G01N 21/85, G01J 3/42, C12M 1/34

(54) **SPECTROSCOPIC SYSTEM AND METHOD FOR ANALYZING COMPOUNDS IN BIOREACTORS**

(30) Priority: 10.01.2025 US 202563743899 P; 09.12.2025 US 202519413959
(71) Applicant: Honeywell International Inc., Charlotte, NC 28202 (US)
(72) Inventor: YEE, Daniel James, Charlotte, 28202 (US); BROWN, Andy Walker, Charlotte, 28202 (US); SHAFAI, Moin S., Charlotte, 28202 (US); WADE, Richard A., Charlotte, 28202 (US); SAI, Bin, Charlotte, 28202 (US)
(74) Representative: Haseltine Lake Kempner LLP

(57) **Abstract**

A system comprising a reactor tank configured to receive a liquid composition via a liquid flow path; a plurality of dual frequency comb spectroscopy (DFCS) configurations, wherein a DFCS configuration of the plurality of DFCS configurations comprises a laser emitter configured to emit laser light through at least a portion of the liquid flow path; and a photodetector configured to (i) receive transmitted light from the laser light passed through the portion of the liquid flow path and (ii) generate a signal output based on one or more absorption or wavelength characteristics of the transmitted light; and a system monitor communicatively coupled to the plurality of DFCS configurations, wherein the system monitor is configured to determine one or more substance concentrations at the portion of the liquid flow path based on the signal output.

## Description

### CROSS REFERENCE TO RELATED APPLICATION

This application claims the priority of U.S. Provisional Application No. 63/743,899, entitled "SPECTROSCOPIC SYSTEM AND METHOD FOR ANALYZING COMPOUNDS IN BIOREACTORS," filed on January 10, 2025, the disclosure of which is hereby incorporated by reference in its entirety.

### BACKGROUND

Manufacturing medications may pose significant challenges, including variability in drug potency, potential contamination, regulatory compliance issues, and the risk of adverse reactions, which may compromise patient safety and treatment efficacy. Additionally, significant amounts of medication may be wasted due to inaccurate dosing and/or inefficient monitoring systems. As such, a need for improved quality control and/r oversight in drug manufacturing practices may be needed. Applicant has identified many technical challenges and difficulties associated with conventional manufacturing of drug compounds.

### BRIEF SUMMARY

Various embodiments described herein relate to components, apparatuses, and systems for performing high-resolution, real-time characterization of chemicals in liquid flow applications. According to some embodiments, a system comprises a reactor tank configured to receive a liquid composition via a liquid flow path; a plurality of dual frequency comb spectroscopy (DFCS) configurations, wherein a DFCS configuration of the plurality of DFCS configurations comprises a laser emitter configured to emit laser light through at least a portion of the liquid flow path; and a photodetector configured to (i) receive transmitted light from the laser light passed through the portion of the liquid flow path and (ii) generate a signal output based on one or more absorption or wavelength characteristics of the transmitted light; and a system monitor communicatively coupled to the plurality of DFCS configurations, wherein the system monitor is configured to determine one or more substance concentrations at the portion of the liquid flow path based on the signal output.

In some embodiments, the DFCS configuration is positioned between a delivery system and the reactor tank. In some embodiments, the DFCS configuration is positioned above a liquid level of the liquid composition within the reactor tank. In some embodiments, the DFCS configuration is positioned below a liquid level of the liquid composition within the reactor tank. In some embodiments, the DFCS configuration is positioned at an output from the reactor tank. In some embodiments, the laser emitter comprises a fiber emitter that is configured to provide a dual frequency comb source that comprises two optical frequency combs configured to emit the laser light at evenly spaced intervals across a spectrum of optical frequencies. In some embodiments, the system monitor is configured to adjust one or more manufacturing parameters based on the measured absorption or wavelength characteristics corresponding to the signal outputs. In some embodiments, the DFCS configuration further comprises a disposable fluid chamber positioned between the laser emitter and the photodetector, wherein the disposable fluid chamber comprises a channel and an optical window.

In some embodiments, (i) the system monitor is configured to determine, using a machine learning algorithm, the one or more substance concentrations; and (ii) the machine learning algorithm comprises one or more of a linear regression algorithm, a logistic regression algorithm, a decision tree algorithm, a support vector machine algorithm, a naive Bayes algorithm, a k-nearest neighbors algorithm, a k-means algorithm, a random forest algorithm, a convolutional neural network (CNN), a recurrent neural network, a generative adversarial network, a transformer, and/or an artificial neural network. In some embodiments, (i) the signal output comprises first spectral data corresponding to the one or more absorption or wavelength characteristics; (ii) the system monitor is configured to determine mixing uniformity within the reactor tank by comparing the first spectral data with second spectral data of another signal output that corresponds to another DFCS configuration of the plurality of DFCS configurations. In some embodiments, the system monitor is configured to determine an impurity based on a difference between spectral data corresponding to the signal output and reference spectral data corresponding to one or more target substances.

According to some embodiments, a computer-implemented method comprises generating, by one or more processors and using a laser emitter, a laser light; receiving, by the one or more processors, a signal output from a photodetector that is configured between the laser emitter and an optical window of a liquid flow path, wherein the photodetector is configured to generate the signal output based on one or more absorption and wavelength characteristics of transmitted light from passing the laser light through the optical window; determining, by the one or more processors, one or more substance concentrations of at least a portion of the liquid flow path based on the signal output; and determining, by the one or more processors, an impurity based on the one or more substance concentrations.

In some embodiments, generating the laser light comprises providing a dual frequency comb source, wherein the dual frequency comb source comprises two optical frequency combs with evenly spaced intervals across a spectrum of optical frequencies. In some embodiments, determining the impurity further comprises determining differences between spectral data from the signal output and reference spectral data that is associated with one or more target substances. In some embodiments, determining the impurity further comprises determining a health of a population of microorganisms within a bioreactor based on the one or more substance concentrations. In some embodiments, the optical window comprises a disposable fluid chamber that is configured in at least the portion of the liquid flow path. In some embodiments, the computer-implemented method further comprises generating, using a plurality of laser emitters including the laser emitter, a plurality of laser light beams at a plurality of locations within a bioreactor system; receiving a plurality of signal outputs from a plurality of photodetectors that respectively correspond to the plurality of laser emitters, wherein the plurality of signal outputs are generated by the plurality of photodetectors based on the plurality of laser light beams from the plurality of laser emitters that respectively correspond to the plurality of photodetectors; and determining mixing uniformity within a reactor tank of the bioreactor system based on spectral data corresponding to the plurality of signal outputs. In some embodiments, the plurality of locations comprises one or more of an input of the liquid flow path, within the reactor tank, or an output of the liquid flow path.

The foregoing illustrative summary, as well as other exemplary objectives and/or advantages of the disclosure, and the manner in which the same are accomplished, are further explained in the following detailed description and its accompanying drawings.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 depicts an example bioreactor in accordance with some embodiments of the present disclosure; and
FIG. 2 depicts a flow diagram of an example process for monitoring a liquid flow path in accordance with some embodiments of the present disclosure.

### DETAILED DESCRIPTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," etc., are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments, or it may be excluded.

As described above, there are many technical challenges and difficulties associated with current practices of manufacturing drug compounds. That is, a need exists for real-time monitoring of drug concentrations, bio-nutrients, and/or other ingredients during manufacturing to ensure consistency and quality. Accurate measurement of drug concentrations may prevent under or overdosing, which may lead to ineffective treatment or adverse effects. Additionally, high costs may be associated with original equipment manufacturer (OEM) losses due to inefficiencies in the manufacturing process.

Various example embodiments of the present disclosure overcome such technical challenges and difficulties in drug manufacturing and provide various technical advancements and improvements. In particular, various embodiments of the present disclosure address challenges in manufacturing medications, such as variability in drug potency, potential contamination, regulatory compliance issues, and the risk of adverse reactions. To do so, systems and methods are provided for real-time monitoring of substance concentrations, such as of drugs and/or medications, during mixing. In some embodiments, spectroscopy, specifically dual frequency comb spectroscopy (DFCS), is used to analyze and/or monitor the mixing of medications and/or biologics in a bioreactor. DFCS may provide high-resolution spectral data, enabling precise real-time characterization (e.g., identification and/or quantification) of substances in complex mixtures, such as drugs and medications, in liquid flow applications. For example, laser light comprising a spectrum of wavelengths at precise intervals may be emitted through one or more liquid flow paths of a bioreactor, allowing for simultaneous analysis of a plurality of spectral lines that may respectively correspond to a plurality of substances.

Various embodiments of the present disclosure may allow for improved real-time monitoring of drug concentrations during manufacturing, ensuring consistency and quality. In particular, DFCS may be used to provide efficiency and customization for specialty mixing of medications and/or biologics in bioreactors. For example, the application of DFCS may improve monitoring and auditing of a reservoir of medicine and/or mixing processes over time to ensure consistency and reduce waste, leading to more efficient manufacturing processes. By continuously monitoring the process inputs, conditions, and outputs with DFCS, a drug manufacturing processes may be controlled in a way that enhances efficiency (e.g., time, cost, yield, batch customization, etc.), and may be done in situ without removing a sample for intermittent testing-something that generates waste and may introduce contaminants into a bioreactor system. By optimizing the use of available medications through precise dosing, waste and costs associated with medication waste and OEM losses may be significantly lowered. In this way, a solution may be provided that ensures stringent quality control and oversight, thereby improving patient safety and/or treatment efficacy.

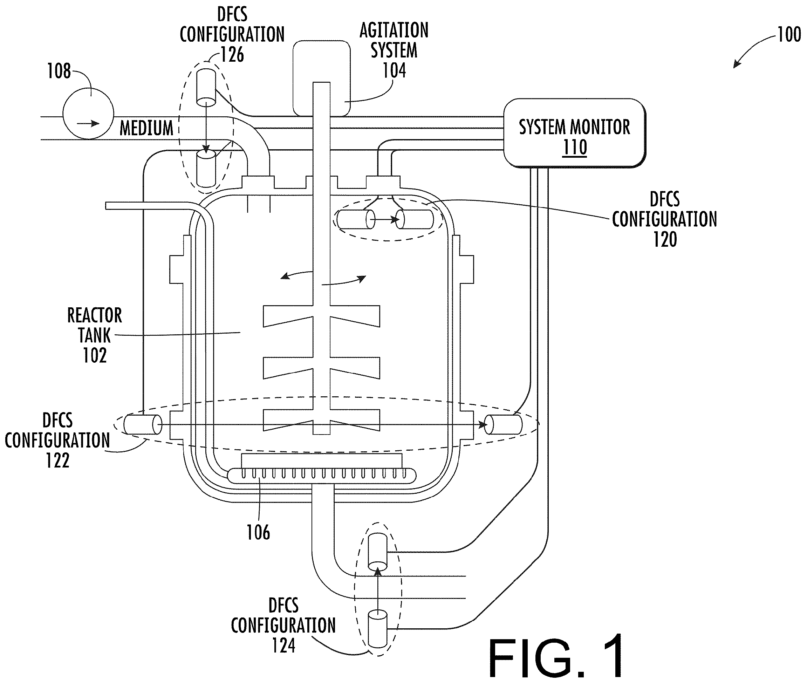

FIG. 1 depicts an example bioreactor 100 in accordance with some embodiments of the present disclosure. The bioreactor 100 is configured, at each of a plurality of DFCS configurations 120, 122, 124, and 126 with a laser emitter and a respectively corresponding photodetector. In some embodiments, a laser emitter may comprise a fiber emitter that provides a dual frequency comb source. The dual frequency comb source may comprise two optical frequency combs that emit laser light at evenly spaced intervals across a spectrum of optical frequencies and/or wavelengths. In some embodiments, a fiber emitter may be configured as compact, cylindrical components that facilitate integration into various locations throughout the bioreactor 100. Within a DFCS configuration of the plurality of DFCS configurations 120, 122, 124, and 126, a laser emitter may emit laser light along an optic path through at least a portion of a liquid flow path and towards a photodetector. In some embodiments, the optic path is configured with a disposable fluid chamber comprising approximately a ±1mm thick channel and a 4mm optical window.

A photodetector of a DFCS configuration of the plurality of DFCS configurations 120, 122, 124, and 126 may be configured to (i) receive and/or capture transmitted light from emitted laser light (from a laser emitter) passed through a liquid composition provided by a liquid flow path, (ii) measure absorption and/or wavelength characteristics (e.g., high-resolution spectral data) of the captured transmitted light, and (iii) generate a signal output based on the measured absorption and/or wavelength characteristics. In some embodiments, an active sampling distance between a laser emitter and a respectively corresponding photodetector may be approximately 1 mm or less. Signal outputs may be generated by one or more photodetectors corresponding to one or more DFCS configurations of the plurality of DFCS configurations 120, 122, 124, and 126 and provided for processing by a system monitor 110 to measure one or more substance concentrations (e.g., concentration of one or more substances), such as of drugs, compounds, biologics, and/or medications, in the liquid flow path.

The system monitor 110 is communicatively coupled to the plurality of DFCS configurations 120, 122, 124, and 126 and receives signal outputs from the photodetectors at each of the plurality of DFCS configurations 120, 122, 124, and 126 simultaneously or sequentially. In some embodiments, the system monitor 110 actively adjusts one or more manufacturing parameters based on the measured absorption and/or wavelength characteristics of the captured transmitted light corresponding to the signal outputs. The system monitor 110 may process the signal outputs using machine learning algorithms to determine concentrations of one or more substances at different locations throughout the bioreactor 100. In some embodiments, the system monitor 110 may compare spectral data from different DFCS configurations to assess mixing uniformity and detect concentration gradients within the reactor tank 102. In some embodiments, the system monitor 110 may be configured to detect impurities by analyzing spectral data from one or more of the plurality of DFCS configurations 120, 122, 124, and 126 and identifying differences from reference spectral data associated with target pharmaceutical compounds or drugs. Such an impurity detection capability may enhance quality control by identifying contamination or unexpected substances during the manufacturing process. The system monitor 110 may implement artificial intelligence and/or machine learning algorithms for analysis (e.g., of a liquid flow path via a signal output generated by a photodetector) that include, but are not limited to, linear regression algorithm, logistic regression algorithm, decision tree algorithm, support vector machine (SVM) algorithm, naive Bayes algorithm, k-nearest neighbors (KNN) algorithm, k-means algorithm, random forest algorithm, recurrent neural network (RNN), a convolutional neural network (CNN), generative adversarial network (GAN), a transformer, artificial neural network, and/or the like, to generate the predictive model.

Accordingly, the plurality of DFCS configurations 120, 122, 124, and 126 may be monitored for real-time (e.g., at a rate between a range of approximately 0.1 Hz to 1 Hz) substance concentrations during mixing and/or manufacturing of compound mixtures within the reactor tank 102, ensuring consistency and quality. For example, a liquid composition in a liquid flow path that is provided from a delivery system 108 and into a reactor tank 102 may be monitored at DFCS configuration 126. The delivery system 108 may comprise one or more of an infusion pump or a syringe. The delivery system 108 may be configured to deliver controlled amounts of pharmaceutical compounds, medications, or biologics into the reactor tank 102 via the liquid flow path. In some embodiments, the delivery system 108 may provide precise flow control to ensure accurate dosing during the manufacturing process.

In some embodiments, the reactor tank 102 may be enclosed within a thermal jacket configured to maintain a controlled temperature environment for the mixing process. The thermal jacket may facilitate temperature regulation of the liquid composition in the liquid flow path within the reactor tank 102, which may be beneficial for maintaining stability of pharmaceutical compounds and biologics during the mixing and manufacturing process.

An agitation system 104 and a submerged agitator 106 may mix a liquid composition in the liquid flow path within the reactor tank 102. The submerged agitator 106 may be positioned at or near the bottom of the reactor tank 102 to provide mixing of the liquid composition. The agitation system 104 may be configured to operate the submerged agitator 106 at various speeds and patterns to achieve desired mixing characteristics for different types of pharmaceutical compounds or biologics.

The reactor tank 102 may be monitored at DFCS configuration 120 (e.g., above liquid level) and 122 (e.g., below liquid level), while a liquid flow path at an output from the reactor tank 102 may be monitored at DFCS configuration 124. In some embodiments, the DFCS configuration 124 may be positioned in an external flow loop that extends from the reactor tank 102. The external flow loop may allow for monitoring of the liquid composition as it exits the reactor tank 102, providing quality control measurements before the final product is collected or transferred to subsequent processing stages. As such, the laser emitter and photodetector configurations at the plurality of DFCS configurations 120, 122, 124, and 126 may provide high-resolution spectral data, enabling precise identification and/or quantification of drugs in complex mixtures.

The plurality of DFCS configurations 120, 122, 124, and 126 are merely provided as example monitoring points and the bioreactor 100 may be monitored with fewer than or greater than the disclosed plurality of DFCS configurations 120, 122, 124, and 126 based on desired parameters and/or measuring locations. For example, in some embodiments, DFCS configurations 122 and 124 may be utilized to monitor the concentration of one or more substances in the liquid composition within the reactor tank 102 and at the output, respectively. In some embodiments, additional DFCS configurations beyond those shown may be incorporated at other strategic locations to provide more comprehensive monitoring coverage.

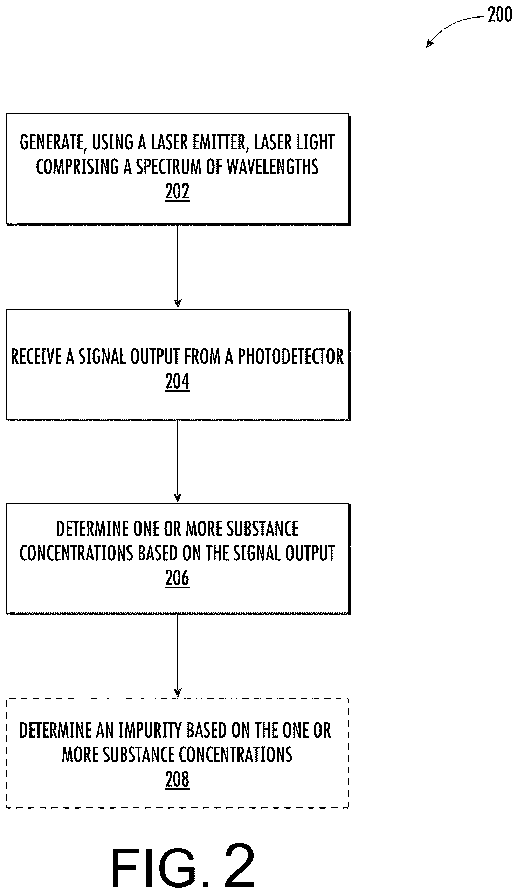

FIG. 2 depicts a flow diagram of an example process 200 for monitoring a liquid flow path in accordance with some example embodiments of the present disclosure. Via the steps/operations of process 200, real-time monitoring and/or quality control in a bioreactor process and/or system (e.g., bioreactor 100) may be provided. In particular, DFCS (e.g., one or more DFCS configurations of the plurality of DFCS configurations 120, 122, 124, and 126) may be used to achieve high-resolution and high-sensitivity measurements of substance concentrations in a bioreactor process and/or system, ensuring accurate dosing and reducing medication waste.

It is noted that each block of a flowchart, and combinations of blocks in the flowchart, may be implemented by various means such as hardware, firmware, circuitry, and/or other devices associated with execution of software including one or more computer program instructions. For example, one or more of the steps/operations described in FIG. 2 may be embodied by computer program instructions, which may be stored by a non-transitory memory of an apparatus (e.g., system monitor 110) employing an embodiment of the present disclosure and executed by a processor component in an apparatus. For example, these computer program instructions may direct the processor component to function in a particular manner, such that the instructions stored in the computer-readable storage memory produce an article of manufacture, the execution of which implements the function specified in the flowchart block(s).

In some embodiments, the process 200 begins at step/operation 202 when, using a laser emitter, laser light comprising a spectrum of wavelengths is generated. A laser emitter may be initiated to provide a dual frequency comb source. The dual frequency comb source may comprise two optical frequency combs with slightly different repetition rates, enabling the generation of laser light at evenly spaced intervals across a spectrum of optical frequencies corresponding to the spectrum of wavelengths. For example, a broad spectrum of wavelengths at precise intervals may be generated, allowing for the simultaneous interrogation of multiple spectral lines corresponding to various drugs. In some embodiments, the spectrum of wavelengths may be selected to correspond to absorption characteristics of target pharmaceutical compounds or biologics being monitored in the liquid flow path.

A laser emitter may be configured to emit the laser light through an optical window of a liquid flow path towards a photodetector. For example, the optical window may comprise a disposable fluid chamber that is configured in at least a portion of a liquid flow path or a reservoir between a drug delivery system (e.g., an infusion pump or a syringe) and a bioreactor (e.g., bioreactor 100). Passing laser light comprising a broad spectrum of wavelengths at precise intervals through the optical window of the liquid flow path may provide transmitted light that may be captured by a photodetector to enable identification and/or quantification in complex mixtures, improving signal resolution and enhancing sensitivity to detect trace concentrations of pharmaceuticals in real time.

In some embodiments, subsequent to step/operation 202, the example process proceeds to step/operation 204, where a signal output is received from a photodetector. The signal output may be generated by a photodetector that is configured between the laser emitter and at least a portion of the optical window. The photodetector may generate the signal output based on absorption and/or wavelength characteristics measured from transmitted light that is received and/or captured by the photodetector. The transmitted light may comprise the laser light emitted from the laser emitter that has passed through the optical window of the liquid flow path. In some embodiments, the signal output may comprise high-resolution spectral data that comprises information about multiple spectral lines. Each spectral line may correspond to a specific pharmaceutical compound, drug, or biologic present in the liquid flow path. In some embodiments, the signal output may be generated at a rate between approximately 0.1 Hz to 1 Hz to provide real-time monitoring capabilities during a manufacturing process.

In some embodiments, subsequent to step/operation 204, the example process proceeds to step/operation 206, where one or more substance concentrations of at least a portion of the liquid flow path are determined based on the signal output. Determining the one or more substance concentrations may comprise analyzing absorption patterns at specific wavelengths that are characteristic of particular pharmaceutical compounds. That is, the measured absorption and/or wavelength characteristics may comprise one or more spectral lines that correspond to one or more specific drugs. The high-resolution spectral data provided by DFCS may enable simultaneous quantification of multiple substances in complex mixtures.

In some embodiments, the signal output is provided to and processed by a machine learning algorithm that is configured to determine concentration of multiple substances (e.g., drugs and/or medication) in the liquid flow path. For example, a machine learning algorithm may be trained on reference spectral data corresponding to known concentrations of target substances. In some embodiments, the machine learning algorithm may comprise one or more of a linear regression algorithm, a logistic regression algorithm, a decision tree algorithm, a SVM algorithm, a naive Bayes algorithm, a KNN algorithm, a k-means algorithm, a random forest algorithm, a RNN, a CNN, a GAN, a transformer, or an artificial neural network.

In some embodiments, subsequent to step/operation 206, the example process proceeds to optional step/operation 208, where an impurity is determined based on the one or more substance concentrations. Determining an impurity may comprise analyzing spectral data (e.g., corresponding to the absorption and/or wavelength characteristics) provided by the signal output by comparing and/or determining differences between the measured spectral data from the signal output and reference spectral data associated with one or more target substances (e.g., compounds, biologics, and/or drugs). Deviations from the reference spectral data may indicate the presence of contaminants, degradation products, or unexpected substances in the liquid flow path. In some embodiments, determining impurity may comprise identifying specific spectral lines that do not correspond to any expected target substances in the liquid flow path. In some embodiments, determining the impurity further comprises determining a health of a population of microorganisms within a bioreactor based on the one or more substance concentrations.

In some embodiments, the process 200 may be repeated continuously and/or at regular intervals during a manufacturing process to provide ongoing real-time monitoring of substance concentrations. The repeated execution of process 200 may enable detection of concentration changes over time, allowing for adjustments to the manufacturing process to maintain desired concentration levels and ensure product quality.

In some embodiments, the process 200 may be executed simultaneously at multiple locations within a bioreactor, such as at multiple DFCS configurations (e.g., DFCS configurations 120, 122, 124, and 126) positioned along different liquid flow paths or at different positions within a reactor tank (e.g., reactor tank 102). The simultaneous execution may provide comprehensive monitoring of substance concentrations throughout the manufacturing system, enabling detection of concentration gradients and assessment of mixing uniformity. For example, simultaneously executing process 200 may comprise generating, using a plurality of laser emitters, a plurality of laser light beams at a plurality of locations within a bioreactor system; receiving a plurality of signal outputs from a plurality of photodetectors that respectively correspond to the plurality of laser emitters, wherein the plurality of signal outputs are generated by the plurality of photodetectors based on the plurality of laser light beams from the plurality of laser emitters that respectively correspond to the plurality of photodetectors; and determining mixing uniformity within a reactor tank of the bioreactor system based on spectral data corresponding to the plurality of signal outputs.

In some embodiments, results from step/operation 206 and/or optional step/operation 208 may be provided for display, logging, or further analysis. For example, one or more alerts and/or notifications may be generated when substance concentrations deviate from target ranges and/or when impurities are detected, enabling prompt corrective action during the manufacturing process.

It is to be understood that the disclosure is not to be limited to the specific embodiments disclosed, and that modifications and other embodiments are intended to be included within the scope of the appended claims. Although specific terms are employed herein, they are used in a generic and descriptive sense only and not for purposes of limitation, unless described otherwise.

## Claims

1. A system comprising:
a reactor tank configured to receive a liquid composition via a liquid flow path;
a plurality of dual frequency comb spectroscopy (DFCS) configurations, wherein a DFCS configuration of the plurality of DFCS configurations comprises:
a laser emitter configured to emit laser light through at least a portion of the liquid flow path; and
a photodetector configured to (i) receive transmitted light from the laser light passed through the portion of the liquid flow path and (ii) generate a signal output based on one or more absorption or wavelength characteristics of the transmitted light; and
a system monitor communicatively coupled to the plurality of DFCS configurations, wherein the system monitor is configured to determine one or more substance concentrations at the portion of the liquid flow path based on the signal output.

2. The system of claim 1, wherein the DFCS configuration is positioned (i) between a delivery system and the reactor tank, (ii) above a liquid level of the liquid composition within the reactor tank, (iii) below a liquid level of the liquid composition within the reactor tank, or (iv) at an output from the reactor tank.

3. The system of claim 1, wherein the laser emitter comprises a fiber emitter that is configured to provide a dual frequency comb source that comprises two optical frequency combs configured to emit the laser light at evenly spaced intervals across a spectrum of optical frequencies.

4. The system of claim 1, wherein the system monitor is configured to adjust one or more manufacturing parameters based on the measured absorption or wavelength characteristics corresponding to the signal outputs.

5. The system of claim 1, wherein the DFCS configuration further comprises a disposable fluid chamber positioned between the laser emitter and the photodetector, wherein the disposable fluid chamber comprises a channel and an optical window.

6. The system of claim 1, wherein:
(i) the system monitor is configured to determine, using a machine learning algorithm, the one or more substance concentrations; and
(ii) the machine learning algorithm comprises one or more of a linear regression algorithm, a logistic regression algorithm, a decision tree algorithm, a support vector machine algorithm, a naive Bayes algorithm, a k-nearest neighbors algorithm, a k-means algorithm, a random forest algorithm, a recurrent neural network, a convolutional neural network, a generative adversarial network, a transformer, or an artificial neural network.

7. The system of claim 1, wherein:
(i) the signal output comprises first spectral data corresponding to the one or more absorption or wavelength characteristics; and
(ii) the system monitor is configured to determine mixing uniformity within the reactor tank by comparing the first spectral data with second spectral data of another signal output that corresponds to another DFCS configuration of the plurality of DFCS configurations.

8. The system of claim 1, wherein the system monitor is configured to determine an impurity based on a difference between spectral data corresponding to the signal output and reference spectral data corresponding to one or more target substances.

9. A computer-implemented method comprising:
generating, by one or more processors and using a laser emitter, a laser light;
receiving, by the one or more processors, a signal output from a photodetector that is configured between the laser emitter and an optical window of a liquid flow path, wherein the photodetector is configured to generate the signal output based on one or more absorption and wavelength characteristics of transmitted light from passing the laser light through the optical window;
determining, by the one or more processors, one or more substance concentrations of at least a portion of the liquid flow path based on the signal output; and
determining, by the one or more processors, an impurity based on the one or more substance concentrations.

10. The computer-implemented method of claim 9, wherein generating the laser light comprises providing a dual frequency comb source, and the dual frequency comb source comprises two optical frequency combs with evenly spaced intervals across a spectrum of optical frequencies.

11. The computer-implemented method of claim 9, wherein determining the impurity further comprises determining differences between spectral data from the signal output and reference spectral data that is associated with one or more target substances.

12. The computer-implemented method of claim 9, wherein determining the impurity further comprises determining a health of a population of microorganisms within a bioreactor based on the one or more substance concentrations.

13. The computer-implemented method of claim 9, wherein the optical window comprises a disposable fluid chamber that is configured in at least the portion of the liquid flow path.

14. The computer-implemented method of claim 9 further comprising:
generating, using a plurality of laser emitters including the laser emitter, a plurality of laser light beams at a plurality of locations within a bioreactor system;
receiving a plurality of signal outputs from a plurality of photodetectors that respectively correspond to the plurality of laser emitters, wherein the plurality of signal outputs are generated by the plurality of photodetectors based on the plurality of laser light beams from the plurality of laser emitters that respectively correspond to the plurality of photodetectors; and
determining mixing uniformity within a reactor tank of the bioreactor system based on spectral data corresponding to the plurality of signal outputs.

15. The computer-implemented method of claim 14 wherein the plurality of locations comprises one or more of an input of the liquid flow path, within the reactor tank, or an output of the liquid flow path.
